# EUROPÄISCHE PATENTSCHRIFT

(11) **EP 0 969 896 B1**
(45) Veröffentlichungstag und Bekanntmachung des Hinweises auf die Patenterteilung: **25.10.2006**
(21) Anmeldenummer: 98913568.6
(22) Anmeldetag: 20.02.1998
(51) Int. Cl.: A61N 1/00

(54) **LERNFÄHIGER SENSOMOTORISCHER ENCODER FÜR NEUROPROTHESEN**
ADAPTIVE SENSO-MOTOR ENCODER FOR NEUROPROSTHESES
CODEUR SENSORI-MOTEUR ADAPTATIF POUR NEUROPROTHESES

(30) Priorität: 21.02.1997 DE 19707046
(43) Veröffentlichungstag der Anmeldung: 12.01.2000
(60) Teilanmeldung: 06001551.8
(73) Patentinhaber: Intelligent Acquisition, LLC, Brookfield, WI 53045-5111 (US)
(72) Erfinder: Eckmiller, Rolf, 53117 Bonn (DE)
(74) Vertreter: Graf von Stosch, Andreas
(86) Internationale Anmeldenummer: PCT/EP1998/000971
(87) Internationale Veröffentlichungsnummer: WO 1998/036793

(56) Entgegenhaltungen:
- US-A- 4 146 029
- US-A- 5 501 703
- US-A- 5 522 863
- ECKMILLER R: "Biology-inspired pulse processing neural networks (BPN) for neurotechnology" ICANN '94., PROCEEDINGS OF INTERNATIONAL CONFERENCE ON ARTIFICIAL NEURAL NETWORKS, SORRENTO, ITALY, 26-29 MAY 1994, Seiten 1329-1334 vol.2, XP002071156 ISBN 3-540-19887-3, 1994, Berlin, Germany, Springer-Verlag, Germany
- ECKMILLER R E: "Concerning the development of retina implants with neural nets" PROGRESS IN NEURAL INFORMATION PROCESSING. PROCEEDINGS OF 1996 INTERNATIONAL CONFERENCE ON NEURAL INFORMATION PROCESSING. ICONIP '96, HONG KONG, 24-27 SEPT. 1996, Seiten 21-28 vol.1, XP002071155 1996, Singapore, Springer-Verlag, Singapore

## Beschreibung

Die Erfindung betrifft einen lernfähigen sensomotorischen Encoder mit den Merkmalen des Oberbegriffs des Anspruchs 1 sowie ein Spinal Implant und ein Cranial Implant.

Es sind mehrere Systeme als Vorläufer von Spinal Implants bekannt, die z.B. bei Querschnittsgelähmten über implantierte, oder durch die Haut wirkende Stimulationskontakte die Steuerung von Funktionen des Harntraktes sowie die Steuerung von Gehbewegungen oder Greifbewegungen bewirken (s. Eckmiller et al., Neurotechnologie Report, 1994 und 1995).

Die gegenwärtig verfügbaren oder in Entwicklung befindlichen Vorläufer von Spinal Implants haben diverse Einschränkungen, z.B. keine Lernfähigkeit, keine funktionelle Mikrokontaktzahl-Erhöhung und keine bidirektionale, wahrnehmungsbasierte Steuerung durch den Implantatträger.

Insbesondere arbeiten die für eine Implantation gegenwärtig entwickelten Mikrokontaktstrukturen sowie Signal- und Energieübertragungssysteme unidirektional vom externen Encoder zum implantierten Stimulator und bieten daher keine Möglichkeit zur laufenden Überwachung der neuronalen Impulsaktivität der stimulierten Neuronen. Dadurch kann die Stimulationspuls-Sequenz nicht an die Spontanaktivität der Neuronen angepaßt werden. Ferner kann die Auslösung von neurobiologischen Impulsen durch Stimulationspulse nicht direkt überwacht werden. Außerdem fehlt eine sichere Impuls-Überwachungsmöglichkeit für eine mögliche zeitliche Abstimmung und Synchronisation der Impulsfolgen mehrerer Neuronen.

Es gibt vereinzelt Ansätze zur Entwicklung von implantierten, bedarfsgesteuerten Wirkstoffapplikatoren, z.B. für Insulin, jedoch existieren bisher keine erfolgreich eingesetzten lernfähigen Cranial Implants. Cranial Implants, die z.B. zur ereignisgetriggerten lokalen Wirkstoffapplikation zur Unterdrückung beginnender Epilepsieanfälle dringend benötigt werden, sind nicht verfügbar.

In der US 4,146,029 wird eine Vorrichtung zur regelmäßigen oder bedarfsgesteuerten Appliktion von Medikamenten am Herzen offenbart. In diesem Stand der Technik wird der Spannungsverlauf des Elektrokardiogramms am Herzmuskel aufgezeichnet, um dessen Tätigkeit bestimmen zu können. Entsprechend wird in der US 4,146,029 die Detektion und Aktorik am Muskel vorgenommen. Aus dem Stand der Technik (US 5,501,703) ist weiterhin bekannt, eine Anordnung von bspw. 9 Elektroden einzusetzen, mit denen insgesamt mehrere 100000 Nervenzellen bzw. - fasern angeregt werden können. Aufgrund der in der US 5,501,703 offenbarten Vorgehensweise ist ersichtlich, dass die globale Beaufschlagung einer sehr großen Region des Nervensystems mit elektrischen Feldern über großflächige Elektroden nicht zur Stimulation einiger weniger oder gar einzelnen Zellen geeignet ist. Es ist aus der US 5,501,703 auch nicht ersichtlich, inwieweit eine Überwachung der Impulsantwort der Nervenzellen vorgesehen sein soll.

Bei Eckmiller et al. (Conceming the development of retina implants with neural nets; Proceedings of 1996 International Conference On Neural Information Processing; ICONIP 1996, 1996 Singapore, Springer-Verlag, Singapore, S. 21-28, Vol. 1) wird ein Retina-Implantat vorgeschlagen, das verschiedene Voraussetzungen erfüllen sollte, wobei die Kommunikation zwischen dem Retina-Encoder und den Mikrokontakten unidirektional erfolgt.

Es ist die Aufgabe der Erfindung, diese Nachteile zu beseitigen und einen lernfähigen, sensomotorischen Encoder zu schaffen, der mit Hilfe neuronaler Netze im Dialog mit dem Implantatträger oder in bidirektionalem Signal- und Datenaustausch von Implantat und kontaktiertem Nervengewebe eine Optimierung der gestörten Funktion des Nervensystems durchführt, die Zahl der selektiv erreichbaren Stimulationsorte funktionell erhöht sowie die neuronale Aktivität einzelner zu stimulierender Neuronen überwacht. Es ist weiter Aufgabe der Erfindung, ein Spinal Implant sowie ein Cranial Implant zu schaffen.

Diese Aufgabe wird von einem Encoder mit den Merkmalen des Anspruchs 1 gelöst. Ferner wird diese Aufgabe, von einem Implantat mit den Merkmalen des Anspruchs 10 und von einem Implantat mit den Merkmalen des Anspruchs 11 gelöst.

Weil der Encoder in bidirektionaler Kopplung mit implantierten Mikrokontakten steht, kann die Überwachung der neuronalen Impulsaktivität einzelner zu stimulierender Neuronen sowie anderer Signale und die Ausführung teilweise autonomer Aktionen realisiert werden. Die Funktionen können durch neuronale Netze selbsttätig oder im Dialog mit dem Implantatträger optimiert werden. Die Zahl der selektiv erreichbaren Stimulationsorte kann funktionell erhöht und die neuronale Aktivität einzelner Neuronen überwacht werden. Die implantierte Struktur kann unter Verwendung geeigneter sensorischer und aktorischer Komponenten sowie eines lernfähigen Kontrollsystems teilweise autonom sensomotorisch agieren. Wesentliche Teile und Verfahren des lernfähigen Informationsverarbeitungssystems werden ferner in unterschiedlichen Kombinationen eingesetzt insbesondere für Spinal Implants in bidirektionalem Kontakt mit dem Rückenmark oder peripheren Nervensystem und für Cranial Implants in bidirektionalem Kontakt mit Strukturen des Zentralnervensystems innerhalb des Schädels.

Ferner wird erstmals ein Encoder vorgeschlagen, der eine Erhöhung der Zahl der selektiv erreichbaren Stimulationsorte funktionell ermöglicht und sich auch später an neue Stimulationsbedingungen anpassen kann. Der hier offenbarte Encoder kann (aufgrund seiner Struktur und Funktion als Gruppe von lernfähigen spatiotemporalen Filtern) neben der Stimulationsfunktion auch eine Überwachung und Auswertung der neuronalen Aktivität zu stimulierender Neuronen leisten.

Die den einzelnen Mikrokontakten zugeordneten spatiotemporalen Filter werden, soweit möglich, individuell im Dialog zwischen Encoder und Implantatträger auf optimale Funktion abgestimmt.

Im Unterschied zu einem Encoder mit starrer Vorverarbeitung, also ohne individuelle Verstellmöglichkeit, werden hier auf der Basis des einzig relevanten Kriteriums, nämlich der erzielten Funktionsverbesserung des gegebenen Bereichs des Nervensystems, die einzelnen spatiotemporalen Filter als separate Encoder-Kanäle justiert. Dieser Vorteil schließt ein, daß spätere Funktionsänderungen, z.B. infolge der Verlagerung von Mikrokontakten durch entsprechende Anpassungen der spatiotemporalen Filterfunktion ausgeglichen werden können. Ein Vorteil der Abstimmung der spatiotemporalen Filterfunktion im Dialog mit dem Implantatträger bzw. einem Bereich seines Nervensystems besteht in der Berücksichtigung von Funktionsaspekten, die nur der jeweilige Implantatträger und nur in impliziter Form, nämlich z. B. durch subjektive Bewertung seiner Wahrnehmung oder durch Bewertung und Funktionsüberwachung seines Nervensystems und deren Verwendung für die Encoderjustierung, in den Optimierungsprozeß einbringen kann.

Die asynchronen Impulsfolgen der einzelnen spatiotemporalen Filterausgänge der zunächst funktionell getrennten Encoder-Kanäle als Stimulationssignale werden vorteilhaft selektive Stimulationsorte im Dialog mit dem Implantatträger unter Berücksichtigung der am Stimulationsort registrierten neuronalen Impulse aufeinander abgestimmt.

Weil vorgesehen ist, daß geeignet ausgewählte oder durch ein Lernverfahren bestimmte Zeitverläufe und Ortsverteilungen der durch Superposition verschiedener, aufeinander abgestimmter Stimulationssignale an mehreren Mikrokontakten bewirkten Feldverteilungen als Stimulationsfoci örtlich und zeitlich selektiv neuronale Impulserregungen auslösen, wird die Zahl der selektiv erreichbaren Stimulationsorte und deren Trennschärfe bzw. Übersprechdämpfung bei einer festen Zahl von implantierten Mikrokontakten funktionell erhöht.

Bei einer gegebenen relativ geringen Zahl implantierter und dauerhaft funktionstüchtiger Mikrokontakte, deren Position relativ zu den Neuronen nicht modifiziert werden kann, ist es von großem Vorteil, funktionell, also durch Erzeugung geeigneter Signale, die Zahl der selektiv erreichbaren Stimulationsorte bzw. Neuronen und damit gleichzeitig die Zahl der separat verfügbaren Encoder-Kanäle bei einer hinreichenden Reserve an spatiotemporalen Filtern zu erhöhen. Dieser Vorteil bewirkt eine Verbesserung der Qualität der jeweils gegebenen Funktion.

Die Steuerung bzw. Linderung von defekten Funktionen des Rükkenmarks oder peripheren Nervensystems mit Hilfe einer teilweise implantierten Neuroprothese in möglichst enger sensorischer und motorischer Kopplung mit dem Implantatträger und unter Verwendung teilweise autonomer sensomotorischer Funktionen der implantierten Struktur wird ermöglicht.

Mit lernfähigen Spinal Implants kann die Qualität der Linderung von neuronalen Funktionsstörungen im Rückenmark oder peripheren Nervensystem wesentlich verbessert und bezüglich diverser Anwendungen erstmals ermöglicht werden.

Es wird Linderung von neuronalen Funktionsstörungen des Zentralnervensystems innerhalb des Schädels insbesondere zur Verminderung unerwünschter sensorischer, motorischer, oder kognitiver Wirkungen für mehrere Patientengruppen der Neurologie oder Psychiatrie mit Hilfe einer implantierten Struktur mit Wirkstoff-Applikator und teilweise autonomen, sensomotorischen Funktionen in Kopplung mit Kontroll- und Wahrnehmungsfunktionen des Implantatträgers ermöglicht.

Mit lernfähigen Cranial Implants wird die Qualität der Linderung von neuronalen Funktionsstörungen im Zentralnervensystem innerhalb des Schädels für diverse Anwendungen erstmals ermöglicht.

Diese vorteilhaften Ausgestaltungen ergeben sich aus den Merkmalen der abhängigen Ansprüche. Ausführungsbeispiele der vorliegenden Erfindung werden im folgenden anhand der Zeichnung beschrieben. Es zeigen:
- Figur 1.:: Ein Ausführungsbeispiel eines Spinal Implants im Bereich des Nervengewebes mit einer Kontrolleinheit und einem durch Kopfbewegungen aktivierten Kommandomodul;
- Figur 2:: eine schematische Darstellung einer implantierten Mikrokontaktstruktur zur Stimulation von nicht unmittelbar kontaktiertem Nervengewebe; sowie
- Figur 3:: eine schematische Darstellung eines Cranial Implants mit den verschiedenen Modulen in einem Blockschaltbild.

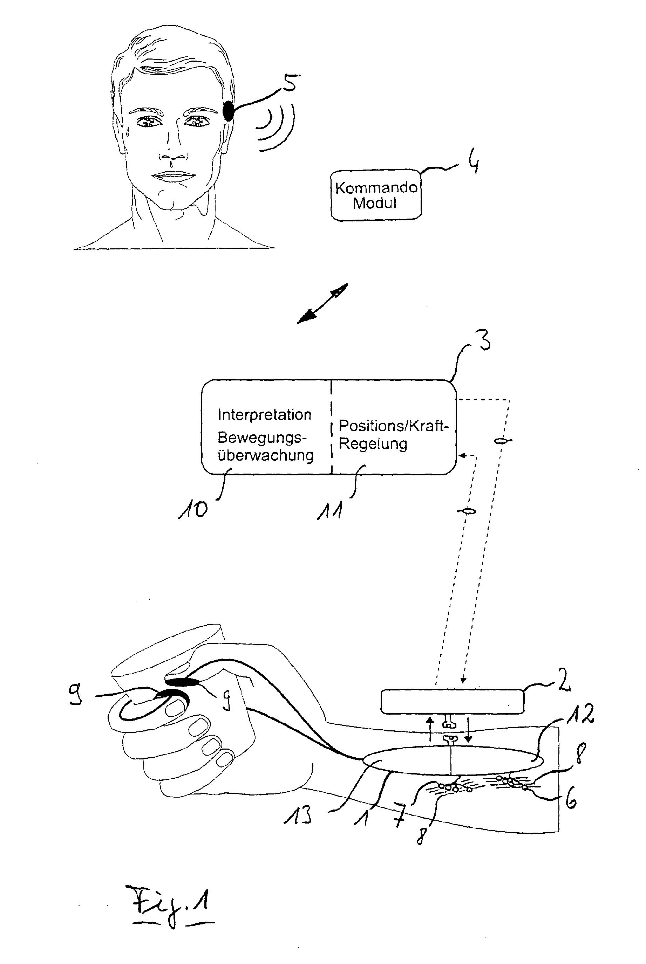

In der Figur 1 ist ein Spinal Implant als Greifprothese veranschaulicht. Diese Prothese kommt zum Einsatz, wenn bei einer Querschnittslähmung Armbewegungen noch durchgeführt werden können, aber Greifbewegungen der Hand ausgefallen sind.

Die Greifprothese umfaßt ein Implantat 1, das mit einem transkutanen Modul 2 kommuniziert. Das Modul 2 wiederum steht in bidirektionaler, drahtloser Verbindung mit einer lernfähigen zentralen Kontrolleinheit 3. Diese Kontrolleinheit empfängt Befehle von einem Kommandomodul 4, das wiederum von einem Kopfbewegungssensor 5 gesteuert wird.

Das Implantat 1 steht über Mikrokontakte 6, 7 in Verbindung mit Nervengewebe 8. Außerdem sind an das Implantat 1 mehrere Sensoren 9 angeschlossen, die im Bereich des Daumenendgliedes und eines weiteren Fingerendgliedes Meßwerte für den Greifvorgang erfassen.

Zur Erzeugung und Steuerung eines Greifvorganges arbeitet das insoweit beschriebene System in der folgenden Weise. Zunächst wird der Benutzer die Hand zu einem Gegenstand bewegen, den er ergreifen will. Dies kann beispielsweise ein Glas sein. Wenn die Hand in der richtigen Position zum Ergreifen des Glases ist, wird über eine bestimmte Kopfbewegung, die in dem Kopfbewegungssensor 5 erkannt wird, der Greifvorgang ausgelöst. Dabei übermittelt der Kopfbewegungssensor 5 seine Signale an das Kommandomodul 4. Dieses generiert geeignete Steuerungsbefehle, die wiederum drahtlos oder per Signalleitung an die Kontrolleinheit 3 übermittelt werden. Dort ist in einem ersten Bereich 10 der Kontrolleinheit der Kommandointerpreter vorgesehen, der auch eine Bewegungsüberwachung leistet. Ein zweiter Bereich 11 der Kontrolleinheit 3 umfaßt die Positions- und Kraftregelung. Das vom Kommandomodul 4 kommende Kommando "zugreifen" wird in der Einheit 10 interpretiert und ein geeignetes Bewegungsstimulationsmuster hierfür bestimmt. Die Einheit 11 der zentralen Kontrolleinheit 3 gibt dann Stellbefehle an das transkutane Modul 2, das wiederum dem Implantat 1 und dort insbesondere einer Stimulatoreinheit 12 den Befehl zur Stimulation der geeigneten Nervenbahnen 8 über die Mikrokontakte 6,7 gibt. Die Nervenbahnen 8 steuern über die elektrische Reizung nun die für den Greifvorgang benötigten Muskeln in der Hand, die sich darauf hin kontrahieren und den Greifvorgang auslösen.

Die Sensoren 9 detektieren nun den um den Gegenstand geschlossenen Griff der Hand, indem sie Informationen über die Position, den ausgeübten Druck und den Schlupf des Gegenstandes ermitteln. Das Ziel eines Greifvorganges ist die Ausübung eines Drucks, der so gering wie möglich ist, um den Gegenstand nicht zu beschädigen (rohes Ei), andererseits aber einen schlupffreien Griff zu gewährleisten, der ein Fallenlassen verhindert.

Die Signale der Sensoren 9 werden an eine Sensoreinheit 13 des Implantats 1 übermittelt. Die empfangenen Signale übermittelt die Sensoreinheit 13 an das transkutane Modul 2, das wiederum nach Signalverarbeitung die ermittelten Werte an die Regeleinheit 11 der Kontrolleinheit 3 liefert, wo die Positions- und Kraftregelung für den Greifvorgang vorgenommen wird. Im einzelnen wird die Erregung der Nervenbahnen 8 über die Mikrokontakte 6 so gesteuert, daß ein optimales Greifergebnis erfolgt. Der Benutzer kann beispielsweise nach dem Ergreifen des Glases über eine andere geeignete Kopfbewegung dem Kopfbewegungssensor 5 und damit dem Kommandomodul 4 die Information geben, daß der Greifvorgang nunmehr selbsttätig aufrechterhalten bleiben soll. Die Kontrolleinheit 3 bewirkt dann diese selbsttätige Regelung des Greifvorganges. Als lernfähige Kontrolleinheit 3 wird die Stimulation über die Mikrokontakte 6,7 im Bereich der Nervenbahnen 8 regelmäßig rückgekoppelt, indem die Aktivität der Nervenbahnen 8 über über die Sensoren 9 in der Sensoreinheit 13 des Implantats 1 erfaßt werden. Die Kontrolleinheit 3 hat diese Lernfähigkeit über ein neuronales Netz integriert, das den Ort, die Stärke und den Zeitverlauf der Stimulation der Nervenbahnen 8 so reguliert, daß möglichst genau diejenigen Nervenbahnen 8 in geeigneter Stärke angeregt werden, die für den Greifvorgang erforderlich sind. So kann nach einer Lernphase ein optimaler Greifvorgang gewährleistet werden.

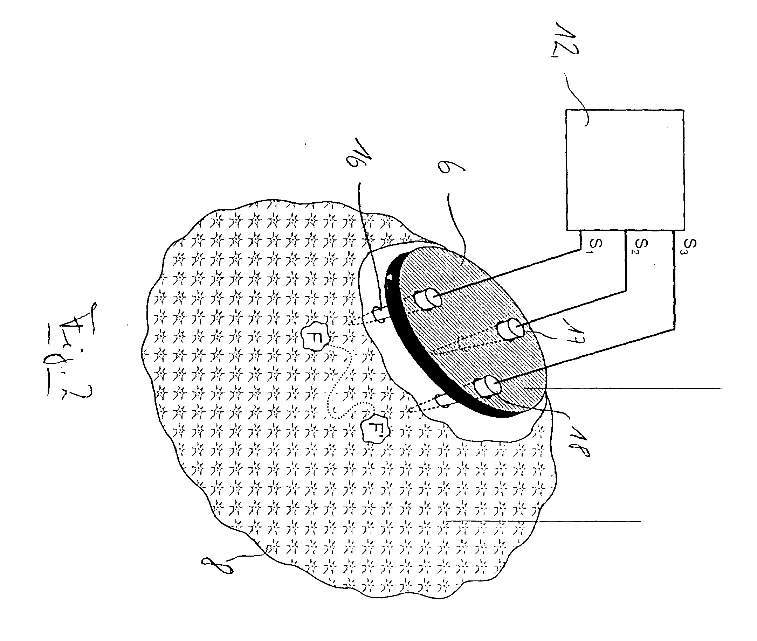

Die Figur 2 zeigt eine veranschaulichte Darstellung von Mikrokontakten 6, die in Nervengewebe 8 eingreifen. Im vorliegenden Beispiel sind drei Mikrokontakte 16, 17, 18 in das Nervengewebe 8 implantiert und dort mehr oder weniger zufällig nahe bestimmten Nervenzellen positioniert. Die Mikrokontaktstruktur 6, 16, 17, 18 ist in jedem Fall wesentlich grober als die Matrix der Nervenzellen 8. Die Mikrokontakte 16, 17, 18 werden über den Stimulator 12 mit Signalen S1, S2 und S3 versorgt.

Um eine gezielte neuronale Anregung zu schaffen, muß beispielsweise ein Stimulationsfokus F erreicht werden, der nicht unmittelbar von einem Mikrokontakt betroffen werden kann. Der Stimulationsfokus F kann aber erreicht werden, indem die Signale S1, S2 und S3 mit unterschiedlichen Stärken, Zeitverläufen und vor allen Dingen zeitlichen Abständen zueinander zu den Elektroden 16, 17 und 18 geleitet werden. Die Überlagerung der erzeugten Signale kann dann so eingerichtet werden, daß das Zusammentreffen der Signale im Bereich des angestrebten Stimulationsfokus F die Reizschwelle einzelner oder weniger Nervenzellen überschreitet, während die Addition der Signalverläufe im übrigen Bereich des Nervengewebes unterhalb der Reizschwelle bleibt.

Durch Änderung der zeitlichen Abfolge und des zeitlichen Signalverlaufs der verschiedenen aufeinander abgestimmten Signale kann auch der Stimulationsfokus von F nach F' verlagert werden. Für den Abgleich dieser Anregungsfunktionen, die einen Stimulationsfokus erreichen, der nicht unmittelbar mit Elektroden in Verbindung steht, ist ein Lernvorgang erforderlich. Da man nicht genau weiß, welcher Stimulationsfokus F, F' für eine bestimmte neurale Anregung angesprochen werden muß, kann die lernfähige sensomotorische Kontrolleinheit nur ein bestimmtes Signalmuster anbieten, das der Implantatträger über eine Sinneswahrnehmung oder eine andere Sensordatenauswertung dann beurteilt. Ein zweites Signalmuster, das gegenüber dem ersten verändert wurde, wird dann ebenfalls daraufhin beurteilt, ob es die gezielte neurale Anregung erreicht oder nicht. Der Benutzer braucht lediglich zu sagen, ob das spätere Signalmuster besser oder schlechter als das vorherige geeignet ist. Mit dem Regelmechanismus eines neuronalen Netzes wird im Laufe des Regelvorgangs eine optimale Signal-Zeitfunktion für die Elektroden 16, 17, 18 zur Anregung des Stimulationsfokus F ermittelt.

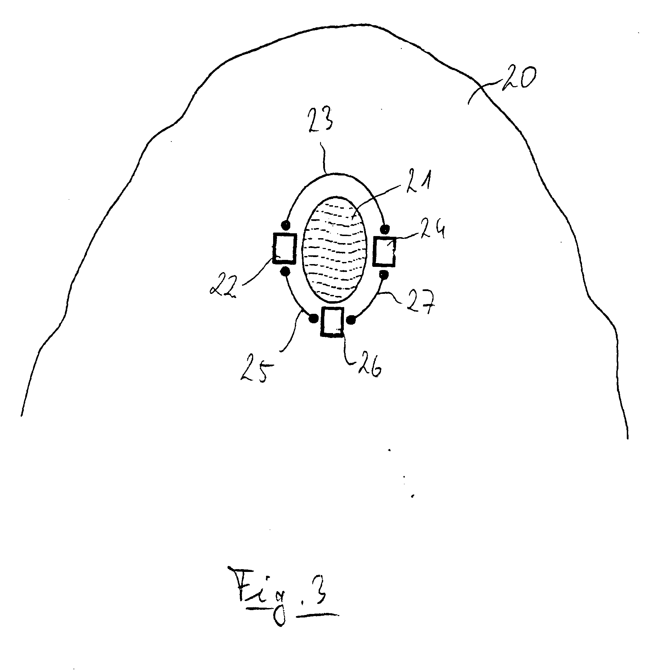

In der Figur 3 ist schließlich ein Cranial Implant zur Überwachung und Beeinflussung einer Nervengeweberegion in der Hirnrinde dargestellt. Ein zu überwachendes Nervengewebe 20 wird im Bereich einer Region 21 mit Mikrokontakten versehen. Die Region 21 kann beispielsweise diejenige Region sein, deren Fehlfunktion zu epileptischen Anfällen führt. Eine Mikrokontaktstruktur 22 umfaßt dabei sowohl die Mikrokontakte zur Stimulation der Nervenzellen als auch Mikrosensoren für die Überwachung der Nervenzellaktivität und weiterer biophysikalischer Parameter. Die Mikrokontaktstruktur 22 steht ihrerseits über einen Signalweg 23 mit einem lernfähigen Prozessor 24 in Verbindung, der seinerseits über ein bidirektional arbeitendes Sende- und Empfangssystem verfügt. Andererseits steht die Mikrokontaktstruktur 22 über einen Signalweg 25 mit einem Wirkstoffspeicher 26 in Verbindung. Der Wirkstoffspeicher 26 ist zur steuerbaren lokalen Freisetzung kleiner Wirkstoffmengen, beispielsweise im Nanoliterbereich, vorbereitet. Schließlich steht der Prozessor 24 über einen weiteren Signalweg 27 mit dem Wirkstoffspeicher 26 unmittelbar in Verbindung.

Die drei Module 22, 24 und 26 des Cranial Implants arbeiten zur Vermeidung von epileptischen Anfällen folgendermaßen zusammen: Die Mikrosensoren überwachen die spontane Nervenzellaktivität in der Region 21 und übermitteln ihre Meßsignale über den Signalweg 23 an den Prozessor 24, der wiederum diese Signale einerseits selbst auswertet und andererseits einen Statusbericht an einen externen Encoder übermittelt. Beim Auftreten von verdächtigen charakteristischen Nervenzellaktivitäten, die sich beispielsweise in einer synchronen, neuronalen Aktivität innerhalb der Region 21 äußern können, erkennt der Prozessor 24 das Entstehen eines Anregungsmusters, das zu einem epileptischen Anfall führen kann. Er kann dann einerseits über den Signalweg 23 an die Mikrokontakte 22 Stimulationspulsfolgen übermitteln, die einer derartigen synchronen, neuronalen Anregung entgegenwirken. Wenn diese rein elektrische Einwirkung nicht ausreicht, kann der Prozessor 24 über den Signalweg 27 dem Wirkstoffspeicher 26 eine Instruktion übermitteln, daß dieser eine genau bemessene Menge eines pharmakologischen Wirkstoffs abgibt, welcher wiederum geeignet ist, die synchrone Nervenzellaktivität auf ein normales Maß abzusenken.

Die Art der Steuerungsvorgänge, ihre zeitliche Abfolge und die Stärke der jeweiligen Einwirkung auf die Nervengeweberegion 21 mittels der Mikrokontaktstruktur 22 und des Wirkstoffspeichers 26 wird über einen externen Encoder eingestellt und optimiert, so daß der Patient im Idealfall von dem künstlichen präventiven Eingriff nichts merkt. Bei physiologischen Veränderungen des Patienten kann die Wirkungsweise des Cranial Implants auch weiter angepaßt werden. Der Encoder hat dabei die wesentlichen Funktionen, die bereits im Zusammenhang mit den Ausführungsbeispielen der Figuren 1 und 2 beschrieben worden sind.

Eine vorteilhafte Ausgestaltung der Struktur und Funktion des Encoders besteht darin, daß ein digitaler Signalprozessor (DSP), beispielsweise das Modell C80 der Fa. Texas Instruments, mit einem Vorverabeitungsmodul, einem Pulssignalsender und Empfänger zur bidirektionalen Kommunikation mit der implantierten Struktur, mehreren Signal-Interfaces insbesondere zur Kommunikation mit der Bewertungseingabe-Einheit und dem externen Überwachungs- und Steuersystem kombiniert ist. Die diversen lernfähigen Informationsverarbeitungsfunktionen sind insbesondere für spatiotemporalen Filter, Dialogmodul und Mustererkennung im DSP mit einer zentralen Kontrolleinheit realisiert und der Benutzer empfängt einerseits Signale als Stimulationspulse oder Sinneswahrnehmungen vom Encoder und sendet andererseits die Bewertungseingabe von biophysikalischen Parametern und neuronaler Aktivität an den Encoder. Ferner besteht ein Ausführungsbeispiel des Encoders darin, daß er aufgrund einer bidirektionalen drahtlosen Signal- und Energieübertragung am Körper oder an einem körperfernen Ort befestigt werden kann. Ferner besteht eine vorteilhafte Ausgestaltung des Encoders darin, daß der spatiotemporale Funktionsraum der spatiotemporalen Filter für den Einsatz als Encoder die informationsverarbeitenden Eigenschaften diverser kontaktierter Neuronenklassen einschließt.

Mit dem Encoder wird eine direkte Verbindung zu einem Teil des Nervensystems hergestellt, welches seinerseits bereits spontan aktiv ist. Es werden also neuronale Impulse von einzelnen Neuronen ohne technische Stimulation generiert. Zur optimalen Anpassung der Stimulationspulsfolgen an die jeweilige Spontanaktivität, zur genauen Bestimmung der Stimulationsparameter für eine sichere und gleichzeitig noch biologisch verträgliche 1:1 Umsetzung von Stimulationspulsen in neuronale Impulse sowie zur besseren Optimierung der zeitlichen Abstimmung und Synchronisation der neuronalen Aktivität mehrerer Neuronen ist die Überwachung der neuronalen Aktivität einzelner zu stimulierender Neuronen von großem Vorteil.

Für die Einstellung der spatiotemporalen Filter des Encoders im Dialog mit dem Benutzer sind die spatiotemporalen Filter als spatiotemporale Filter realisiert, deren spatiale und temporale Funktionsparameter in einem zur Annäherung der jeweiligen informationsverarbeitenden Eigenschaften von Neuronen in einem hinreichend großen Funktionsraum modifiziert werden, nämlich durch an geeigneten Stellen in den Filteralgorithmen plazierte, extern zugängliche Parameteraustauschpunkte. Im Falle eines Lernverfahrens für die spatiotemporalen Filter kann dadurch erfolgen, daß ein Mensch als gesunde Person oder Implantatträger in einem wahrnehmungsbasierten Dialog mit dem Encoder den Wahrnehmungsvergleich zwischen Soll- und Ist-Muster z.B. durch eine, als Zeile von mehreren Schiebereglern gestaltete oder in bestimmten Kopfbewegungen codierte Bewertungseingabeeinheit an ein technisches Neuronales Netz mit nichtüberwachter Lernregel mitteilt und daß das Neuronale Netz den nächsten Parametervektor für das spatiotemporale Filter sowie das nächste Sollmuster festlegt, mit dem Ziel der Verringerung der wahrgenommenen Musterdifferenz im nächsten Dialogschritt. Eine vorteilhafte Ausgestaltung der Suche nach optimalen Parametervektoren für die spatiotemporalen Filter besteht darin, daß in dem Dialogmodul entweder von einem neuronalen Netz mit nichtüberwachtem Lernen jeweils Parametervektoren erzeugt werden, die für ein gegebenes präsentiertes Muster zu einer bestimmten Wahrnehmung führen und entsprechend subjektiv bewertet werden, oder daß in dem Dialogmodul ein anderes Parametereinstellungssystem Sequenzen von Parametervektoren zur virtuellen Bewegung im Funktionsraum des spatiotemporalen Filters z.B. als kontinuierliche Trajektorien nach Art von Scanning oder Sweepverläufen, oder als regellose Folgen, oder als Folgen von neurophysiologisch besonders typischen Filterfunktionen verwendet wird und der Benutzer während dieser in einem geeigneten Zeittakt ablaufenden Sequenz gelegentlich aus dem Zusammenwirken des gegebenen Musters, des nachgeschalteten Vorverarbeitungsmoduls, des nachgeschalteten spatiotemporalen Filters und dem über den zugehörigen Mikrokontakt angekoppelten Teil des Nervensystems verursachte "sinnvolle" Wahrnehmungen meldet und daß anschließend in dem so gefundenen Bereich des Filterfunktionsraumes eine genauere Parameteroptimierung wahrnehmungsbasiert durchgeführt werden kann.

Ein Ausführungsbeispiel der Erzeugung asynchroner Impulsfolgen besteht darin, daß die Ausgangssignale der einzelnen spatiotemporalen Filter durch geeignete Umsetzer-Algorithmen von den quasikontinuierlichen Zeitfunktionen der spatiotemporalen Filter in asynchrone Impulsfolgen entsprechend der Aktivität der Neuronen gewandelt werden und daß Impulsfolgen-Zeitverläufe sowie Zeitpunkte des Auftretens einzelner Impulse durch variable Zeitverzögerungsglieder in der Dialogphase verstellt werden können.

Die zeitliche Kopplung der von mehreren spatiotemporalen Filtern des Encoders zur Auslösung von Nervenzell-Impulsen erzeugten asynchronen Impulsfolgen kann dadurch erfolgen, daß die Sendezeitpunkte der einzelnen Impulssignale durch steuerbare Zeitverzögerungsglieder so variiert werden, daß sich eine zeitliche Kopplung bis hin zum genau synchronen Auftreten ergibt, daß die Variation der Zeitverzögerung vom Implantatträger gesteuert wird, oder im Dialog wahrnehmungsbasiert über ein neuronales Netz erfolgt, oder extern gesteuert wird, daß die Wahl der zeitlich zu koppelnden Impulsgruppen sowohl vom spatiotemporalen Filter kommende, als auch im Interface registrierte Impulse berücksichtigen kann und daß angesichts der sehr unterschiedlichen momentanen Impulsraten der verschiedenen spatiotemporalen Filter geeignete Kriterien für die Beteiligung einzelner Impulse an zu koppelnden Impulsgruppen festgelegt werden.

Die funktionelle Erhöhung der Zahl und Trennschärfe der selektiv erreichbaren Stimulationsorte bei einer gegebenen Zahl von stationär implantierten Mikrokontakten kann dadurch erfolgen, daß die Impulssignale von einem gegebenen spatiotemporalen Filter an mehrere, örtlich benachbarte Mikrokontakte geleitet werden, daß die aufgrund der Encoderbefehle genau für jeden Mikrokontakt festgelegten und entsprechend im Interface bezüglich Stromamplitude, Polarität und Phasenlage decodierten Stimulations-Zeitfunktionen charakteristische Zeitverläufe des elektromagnetischen Feldes im Bereich der zu stimulierenden Neuronen bewirken, daß diese durch Superposition aufeinander abgestimmter Stimulationssignale an mehreren Mikrokontakten bewirkten Feldverteilungen örtlich und zeitlich selektiv neuronale Impulserregungen auslösen, daß die selektiven Stimulationsorte durch geeignete Variation der superponierten Stimulationssignale schnell gewechselt werden können und daß die entsprechende Variation diversen Parameter der aufeinander abgestimmten Stimulationssignale im wahrnehmungsbasierten Dialog mit dem Implantatträger über ein neuronales Netz, oder andere Signalvariationsverfahren zur Bestimmung möglichst vieler, zu selektiver und trennscharfer neuronaler Erregung führender, Stimulationsorte erfolgt. Ferner kann durch Vergleich registrierter neuronaler Impulse mit den Stimulationssignalen die Optimierung der Stimulations-Zeitfunktionen bezüglich angestrebter Einzelzellselektivität und dauerhafter Bioverträglichkeit verbessert werden.

Ein Ausführungsbeispiel des Überwachungssystems für eine teilweise sensomotorisch autonom agierende implantierte Struktur des Encoders besteht darin, daß die implantierten Mikrokontakte sowohl zur Stimulation, als auch zur Registrierung neuronaler Impulse verwendet, daß die registrierten Impulse und weiteren physikalischen oder chemischen Signale von der implantierten Struktur durch geeignete Vorverstärker und optische, oder elektromagnetische Sender an den Encoder gemeldet und daß dort die registrierten neuronalen Signale für verschiedene Zwecke der Encoderfunktion weiterverarbeitet werden.

Ein Ausführungsbeispiel von teilweise autonomen sensomotorischen Aktionen der implantierten Struktur besteht darin, daß unter Verwendung von in der implantierten Struktur verfügbaren diversen Sensoren physikalischer oder chemischer Werte, von diversen Aktoren, wie z.B. elektrischen Stimulationselektroden, mechanischen Mikro-Aktoren, chemischen Wirkstoffapplikatoren, oder thermisch wirkenden Sonden für heilende oder mikrochirurgische Zwecke, von Strukturen zur Durchführung chemischer Analysen und Prozesse innerhalb der implantierten Struktur und von einem teilweise neuronalen, lernfähigen Kontrollsystem in Kommunikation mit dem Encoder teilweise autonom diverse sensomotorische Aktionen, also z.B. auf soeben detektierte Sensordaten rasch reagierende Einwirkungen auf das lokale Gewebe ausgeführt werden können. Hierbei kann die bidirektionale Kopplung mit dem Encoder als Steuerung oder Regelung genutzt werden.

Ein Ausführungsbeispiel des lernfähigen spinalen Implantats z.B. für Querschnittsgelähmte zur neuronalen Modulation des Harntraktes, zur Steuerung von Greif- oder Gehbewegungen, oder zur Reduzierung von Phantomschmerzen nach Amputationen besteht darin, daß sich die implantierte Mikrokontaktstruktur im Rückenmark, peripheren Nervensystem, oder in Muskelgruppen befindet, daß der externe lernfähige Encoder in bidirektionaler Kommunikation mit der implantierten Struktur als portable Einheit am Körper des Benutzers getragen wird, mit dem Benutzer zum Signalempfang sowie zur Signalsendung in bidirektionaler Kommunikation steht und weitgehend autonom funktioniert, oder vom Benutzer durch Manipulation oder z.B. durch Kopf- oder Augenbewegungen gesteuert werden kann. Ein Ausführungsbeispiel der implantierten Struktur besteht darin, daß teilweise autonome sensomotorische Aktionen, wie z.B. eine bedarfsgesteuerte Applikation von Wachstumshormon, oder thermische Einwirkung ausgeführt werden.

Ein Ausführungsbeispiel des lernfähigen Cranial Implants z.B. als bedarfsgetriggerter lokaler Wirkstoffapplikator für Epilepsiepatienten, Parkinsonpatienten, oder Psychiatriepatienten besteht darin, daß sich die implantierte Struktur einschließlich lokaler Detektoren physikalischer, neurophysiologischer, sowie Ionen-, Molekül- und Wirkstoffkonzentrationen sowie Wirkstoffdepots mit einfacher Nachfüllmöglichkeit von außen, lokalen Wirkstoffgebern mit Steuerung und lernfähigem Informationsverarbeitungsmodul innerhalb des Schädels befindet und mit einem externen Encoder bidirektional kommuniziert, daß ein medizinisch und technologisch besonders qualifiziertes Team nach informierter Zustimmung des Patienten, wie z.B. vor der Entscheidung über mikrochirurgische Eingriffe durch implantierte thermisch wirkende Sonden, die einzelnen Funktionen nicht nur überwacht, sondern auch steuert und daß die einzelnen Funktionen vom Patienten im wahrnehmungsbasierten Dialog optimiert und überwacht werden können. Ein Ausführungsbeispiel der implantierten Struktur besteht darin, daß teilweise autonome sensomotorische Aktionen, wie z.B. eine bedarfsgesteuerte Applikation von Wachstumshormonen, oder lokal fehlenden Neurotransmittersubstanzen für die Funktion von Synapsen, also der am Lern- und Gedächtnisprozeß wesentlich beteiligten biologischen Kontakte zwischen Nervenzellen sowie z.B. die bedarfsgesteuerte Unterdrückung epileptischer Anfälle oder thermische Einwirkung ausgeführt werden.

## Patentansprüche

1. Lernfähiger, sensomotorischer Encoder für eine Neuroprothese im zentralen Nervensystem innerhalb des Schädels, des Rückenmarks oder im peripheren Nervensystem, mit einer zentralen Kontrolleinheit (3) für Signalverarbeitungsfunktionen, Überwachungsfunktionen, Steuerungsfunktionen und/oder externe Eingriffsfunktionen, sowie mit einer Gruppe von adaptiven spatiotemporalen Filtern für sensorische und/oder motorische Funktionen, **dadurch gekennzeichnet, daß** eine bidirektionale Schnittstelle (2) zur Kopplung des Encoders mit einer implantierbaren Mikrostruktur (6,7) zur Stimulation von Nerven- oder Gliagewebe einerseits sowie zur Funktionsüberwachung von Hirnfunktionen andererseits vorgesehen ist.

2. Vorrichtung nach Anspruch 1, **dadurch gekennzeichnet, daß** die Kontrolleinheit (3) lernfähig und zur Steuerung einer zumindest teilweise autonom sensomotorisch agierenden implantierten Struktur (1,6,7;22,24,26) vorgesehen ist.

3. Vorrichtung nach einem der vorhergehenden Ansprüche, **dadurch gekennzeichnet, daß** mittels der Steuerungsfunktion ein Wirkstoff oder eine Energieform am Ort der implantierten Struktur (22,24,26) applizierbar ist.

4. Vorrichtung nach einem der vorhergehenden Ansprüche, **dadurch gekennzeichnet, daß** die Überwachungsfunktionen die Aktivität von Neuronen oder Gliazellen sowie Ionen-, Molekül- oder Wirkstoffkonzentrationen erfassen.

5. Vorrichtung nach einem der vorhergehenden Ansprüche, **dadurch gekennzeichnet, daß** zur funktionellen Erhöhung der Zahl und Trennschärfe der selektiv erreichbaren Stimulationsorte bei einer gegebenen Zahl von stationär implantierten Mikrokontakten (6) die Impulssignale (S₁, S₂, S₃) sowohl an einzelne als auch an mehrere, örtlich benachbarte Mikrokontakte (16, 17, 18) geleitet werden können, daß die aufgrund der Encoderbefehle genau für jeden Mikrokontakt (16, 17, 18) individuell festgelegten und entsprechend im implantierten Interface (12) insbesondere bezüglich Zeitverlauf, Stromstärke, Polarität und Phasenlage decodierten Stimulations-Zeitfunktionen ihrerseits charakteristische Zeitverläufe und lokale Ortsverteilungen des elektromagnetischen Feldes im Bereich der zu stimulierenden Neuronen bewirken.

6. Vorrichtung nach einem der vorhergehenden Ansprüche, **dadurch gekennzeichnet, daß** die ausgewählten oder durch ein Lernverfahren bestimmten Zeitverläufe und Ortsverteilungen der durch Superposition verschiedener aufeinander abgestimmter Stimulationssignale an mehreren Mikrokontakten (16, 17, 18) bewirkten Feldverteilungen als Stimulationsfoci (F, F') örtlich und zeitlich selektiv neuronale Impulserregungen auslösen.

7. Vorrichtung nach einem der vorhergehenden Ansprüche, **dadurch gekennzeichnet, daß** die selektiven Stimulationsorte (F,F') durch geeignete Variation der superponierten Stimulationssignale (S₁,S₂,S₃) zur örtlichen Verlagerung des Stimulationsfocus (F,F') schnell gewechselt werden können.

8. Vorrichtung nach einem der vorhergehenden Ansprüche, **dadurch gekennzeichnet, daß** ein Überwachungssystem für eine teilweise sensomotorisch agierende implantierte Struktur für die Überwachung und Bewertung diverser registrierter Signale sowie die Kontrolle von am Implantatort erfolgenden Einwirkungen durch die mit dem Encoder bidirektional kommunizierende implantierte Struktur (1,6,7;22,24,26) vorgesehen ist, wobei die implantierte Struktur (1,6,7;22,24,26) mit diversen Sensoren(9,22), chemischen Analyse- und Reaktionsprozessen sowie mit Aktoren (26) für mechanische, elektrische, anderweitige physikalische sowie chemische lokale Einwirkungen am Implantatort und mit einer lernfähigen sensomotorischen Steuerung (1,24) in Kommunikation mit dem Encoder ausgestattet ist.

9. Vorrichtung nach einem der vorhergehenden Ansprüche, **dadurch gekennzeichnet, daß** die implantierten Mikrokontakte (6,7,22) über eine bidirektionale Kommunikation sowohl zur Stimulation als auch zur Registrierung neuronaler Impulse verwendbar sind und/oder daß zusätzlich Mikrokontakte zur Registrierung von neuronaler Aktivität sowie von physikalischen Parametern, wie z.B. Druck oder Temperatur und von Ionen- oder Molekülkonzentrationen in der implantierten Struktur (1, 6, 7;22, 24, 26) verfügbar sind.

10. Lernfähiges Spinal Implant zur Linderung von neuronalen Funktionsstörungen im Rückenmark oder peripheren Nervensystem insbesondere in Bezug auf die Extremitätenmotorik, den Urogenitaltrakt und unerwünschte Schmerzempfindungen oder Phantomwahrnehmungen, mit einem lernfähigen Encoder, einer teilweise autonom sensomotorisch agierenden implantierten Struktur (1,6) und einem bidirektionalen Signal- und Energieübertragungssystem (2,12,13), wobei der Encoder in bidirektionalem Kontakt mit Teilen des Rückenmarks, des peripheren Nervensystems oder von Muskelgruppen steht.

11. Lernfähiges Cranial Implant zur Linderung von neuronalen Funktionsstörungen des Zentralnervensystems mit unerwünschten sensorischen, motorischen oder kognitiven Wirkungen einschließlich Gedächtnisstörungen und Persönlichkeitsveränderungen, mit einem lernfähigen Encoder, mit einer zentralen Kontrolleinheit für Signalverarbeitungsfunktionen, Überwachungsfunktionen, Steuerungsfunktionen und/oder externen Eingriffsfunktionen einer teilweise autonomen, sensomotorisch agierenden implantierten Struktur (22,24,26) mit Mikrokontakten (16, 17, 18, 22), Detektoren, Wirkstoffdepot und Wirkstoffapplikator (26), wobei der Encoder über ein Signal- und Energieübertragungssystem (24) in bidirektionalem Kontakt mit Hirnregionen (21) innerhalb des Schädels steht.

12. Vorrichtung nach einem der vorhergehenden Ansprüche, **dadurch gekennzeichnet, daß** ein Überwachungssystem teilweise autonom durch ein dort vorhandenes lernfähiges Steuerungssystem eine Bewertung der am Implantatort registrierten Signale vorgenimmt und in physikalische oder chemische lokale Einwirkungsbefehle umsetzt.

13. Vorrichtung nach einem der vorhergehenden Ansprüche, **dadurch gekennzeichnet, daß** entsprechende Funktionen des lernfähigen Encoders (3) zur wahrnehmungsbasierten, automatischen oder extern gesteuerten Funktionsanpassung sowohl sensorischer spatiotemporaler Filter zur Empfindungsoptimierung als auch motorischer spatiotemporaler Filter, oder anderer Steuerungsmodule zur Bewegungsverlaufsoptimierung, bedarfsgesteuerten Wirkstoffapplikation, oder mikrochirurgischen, oder heilenden Einwirkung verwendet werden.

14. Vorrichtung nach einem der vorhergehenden Ansprüche, **dadurch gekennzeichnet, daß** bei Spinal Implants Meßsysteme (5) für Augen- und Kopfbewegungen oder andere Artikulationen für Steuerungszwecke von Implantatfunktionen und für Meldezwecke der Intentionen oder Wahrnehmungen des Implantatträgers vorhanden sind und daß entsprechende Verfahren zur Wahl verschiedener Funktionsabläufe durch eine mitgeführte Befehlseingabeeinheit sowie zur laufenden Übermittlung ausgewählter Zustandsparameter bezüglich des Implantatträgers und des Implantats mit Hilfe eines geeigneten, mitgeführten Signalgebers an ein geeignetes Sinnesorgan, z.B. den Tastsinn, oder einen Betreuer verwendet und dem jeweiligen Patienten angepaßt eingesetzt werden können.

15. Vorrichtung nach einem der vorhergehenden Ansprüche, **dadurch gekennzeichnet, daß** ein lernfähiges Steuerungssystem zur Bewertung der am Implantatort registrierten Signale einschließlich chemischer Analysen und Erzeugung von chemischen Reaktionen innerhalb der implantierten Struktur vorgesehen ist, und daß die Bewertung in physikalische oder chemische lokale Einwirkungsbefehle, wie z.B. lokale Elektrostimulation, Wirkstoffapplikation oder thermische Einwirkung für chirurgische oder heilende Zwecke umgesetzt wird.

16. Vorrichtung nach einem der vorhergehenden Ansprüche, **dadurch gekennzeichnet, daß** implantierte lokale Wirkstoffdepots (26) in Abhängigkeit von einem über geeignete implantierte Detektoren(22), beispielsweise für neuronale oder Gliazellenaktivität, physikalische Parameter sowie Ionen-, Molekül-, oder Wirkstoffkonzentrationen, erfaßten und in einer Lernphase bezüglich des Handlungsbedarfes bewerteten, pathophysiologischen Zustandsraum zur lokal begrenzten und zeitlich synchronisierten Ausschüttung bemessener Wirkstoffmengen angesteuert werden und daß Teilfunktionen dieser lokalen zustandsbasierten Wirkstoffgabe autonom in der implantierten Struktur oder in bidirektionaler Kommunikation mit dem Encoder außerhalb des Schädels, direkt vom Implantatträger und/oder von einem Betreuer gesteuert werden können.

## Claims

1. An adaptive, sensory-motor encoder for a neuroprosthesis in the central nervous system within the cranium, the spinal cord or in the peripheral nervous system and equipped with a central control unit (3) for signal processing functions, monitoring functions, checking functions, and or external interventional functions as well as with a group of adaptive spatio-temporal filters for sensory and/or motor functions wherein a bi-directional interface (2) for the coupling of the encoder with an implanted microstructure (6, 7) is provided for stimulation of nerve or glial tissue and for function monitoring of brain function.

2. An encoder according to Claim 1, wherein the control unit (3) is adaptive and is provided for the purpose of controlling an at least quasi-autonomously operating sensory-motor implanted structure (1, 6, 7; 22, 24, 26).

3. An encoder according to any of preceding claims, wherein by means of the checking function, an active substance or a form of energy can be applied at the site of the implanted structure (22, 24, 26).

4. An encoder according to any of preceding claims, wherein the monitoring functions pick up the activity of neurons or glial cells and ionic, molecular, or active substance concentrations.

5. An encoder according to any of preceding claims, wherein for the purpose of functional increase of the number and definition of the selectively addressable stimulation sites with a given number of stationary implanted micro-contacts (6) the impulse signals (S1, S2, S3) can be conducted both at single and at several, locally adjacent micro-contacts (16, 17, 18) that the stimulation-time functions - based on the encoder commands individually established precisely for each micro-contact (16, 17, 18) and decoded corresponding particularly with respect to time course, current strength, polarity and phase length in the implanted interface (12) - effect characteristic time courses and local site distributions of the electromagnetic field in the area of the neurons to be stimulated.

6. An encoder according to any of preceding claims, wherein the time courses and local distributions, selected or determined by an adaptive process of filed distributions effected by superposition of various reciprocally tuned stimulation signals at several micro-sensors (16, 17, 18) trigger local and temporally selective neural impulse stimulations as stimulation foci (F, F').

7. An encoder according to any of preceding claims, wherein the selective stimulation sites (F, F') can be rapidly changed by appropriate variation of the superpositioned stimulation signals (S1, S2, S3) for the purpose of local shift of the stimulation focus (F, F').

8. An encoder according to any of preceding claims, wherein a monitoring system is provided for a partially sensory-motor functioning implanted structure for monitoring and evaluation of diverse recorded signals and the control of effects at the implant site by the implanted structure (1, 6, 7; 22, 24, 26) that is in bi-directional communication with the encoder, whereby the implanted structure (1, 6, 7; 22, 24, 26) is equipped with diverse sensors (9, 22), chemical analysis and reaction processes, and with actors (26) for mechanical, electrical, other physical and chemical local effects at the implant site and further equipped with an adaptive sensory-motor checking system (1, 24) in communication with the encoder.

9. An encoder according to any of preceding claims, wherein the implanted micro-contacts (6, 7, 22) are implementable via a bi-directional communication both for stimulation and for recording of neural impulses and/or wherein additional micro-contacts are available for recording neural activity and recording physical parameters such as, for example, pressure or temperature and of ionic or molecular concentrations in the implanted structure (1, 6, 7; 22, 24, 26).

10. An adaptive spinal implant for alleviation of neural functional impairment in the spinal cord or peripheral nervous system, in particularly with respect to motor aspects of the extremities, the urogenital tracts and undesired pain sensations or phantom perceptions, equipped with an adaptive encoder, a partially autonomously sensory-motorically functioning implanted structure (1, 6) and a bi-directional signal and energy transfer system (2, 12, 13), whereby the encoder is in bi-directional contact with parts of the spinal cord, the peripheral nervous system or muscle groups.

11. An adaptive cranial implant apparatus for alleviation of neural functional impairments of the central nervous system with undesired sensory, motor, or cognitive effects, including memory defects and personality changes, equipped with an adaptive encoder having a central control unit for signal processing functions, monitoring functions, checking functions and/or external intervention functions of a partially autonomously sensory-motorically functioning implanted structure (22, 24, 26) having microcontacts (16, 17, 18, 22), detectors, active substance depot, and active substance administration (26), whereby the encoder is in bi-directional contact with regions of the brain (21) via a signal and energy transfer system (24).

12. An apparatus according to any of preceding claims, wherein a partially autonomous monitoring system undertakes an evaluation of the signals recorded at the implant site by using a locally available adaptive checking system and converts them into physical or chemical local action commands.

13. An apparatus according to any of preceding claims, wherein corresponding functions of the adaptive encoder (3) are used for perception-based, automatic or externally checked function adaptation as well as sensory spatio-temporal filters for sensation optimization and motor spatio-temporal filters, or other checking modules for optimization of the progress of movement, need-driven administration of active substances, or micro-surgical or curative effects.

14. An apparatus according to any of preceding claims, wherein in the case of spinal implants, measurement systems (5) for eye and head movements or other articulations are available for the purposes of checking/controlling implant functions and for purposes of reporting the intentions or perceptions of the implant carrier and wherein the corresponding processes can be used for selection of different function proceedings by a portable command input unit as well as for ongoing transmittal of selected conditional parameters with respect to the implant carrier and the implant with the aid of a suitable, portable signal generator to a sensory organ, for example, the tactile sense, or to an attendant and can be adapted to the patient and implemented.

15. An apparatus according to any of preceding claims, an adaptive checking system is provided for evaluation of the signals recorded at the implant site, including chemical analysis and production of chemical reactions within the implanted structure and that the evaluation is converted into physical or chemical local action commands such as, for example, local electrical stimulation, administration of active substances or thermal effects for surgical or curative purposes.

16. An apparatus according to any of preceding claims, wherein the implanted local active substance depots (26) are addressed in dependence on an pathophysiological condition or status space - in an adaptive phase evaluated with respect to necessity of action - captured by suitable implanted detectors (22), for example for neural or glial cell activity, physical parameters and ionic, molecular, or active substance concentrations for locally limited and temporally synchronized dispensing of metered quantities of active substance and that subfunctions of this locally status-based dosing of active substance can be controlled autonomously in the implanted structure or in bi-directional communication with the encoder external to the cranium, directly by the implant carrier and/or by an attendant.

## Revendications

1. Codeur sensori-moteur adaptatif pour une neuroprothèse placée dans le système nerveux central à l'intérieur de la boîte crânienne, de la moelle épinière ou du système nerveux périphérique, avec unité centrale de contrôle (3) des fonctions de traitement de signaux, pour des fonctions de surveillance, pour des fonctions de commande et/ou pour des fonctions d'intervention externe, ainsi qu'avec un groupe de filtres spatio-temporels adaptatifs pour des fonctions sensorielles et/ou motrices, **caractérisé en ce qu'**est prévue une interface bidirectionnelle (2) pour le couplage du codeur avec une microstructure implantable (6, 7) servant d'une part à stimuler des tissus nerveux et des tissus gliaux, ainsi que d'autre part à surveiller des fonctions cérébrales.

2. Dispositif selon la revendication 1, **caractérisé en ce que** l'unité de contrôle (3) est adaptative et est prévue pour la commande d'une structure implantée (1, 6, 7 ; 22, 24, 26) qui agit au moins partiellement de façon autonome et de manière sensorimotrice.

3. Dispositif selon l'une quelconque des revendications précédentes, **caractérisé en ce qu'**une substance active ou une forme d'énergie est applicable sur le lieu de la structure implantée (22, 24, 26) au moyen de la fonction de commande.

4. Dispositif selon l'une quelconque des revendications précédentes, **caractérisé en ce que** les fonctions de surveillance saisissent l'activité de neurones ou de cellules gliales, ainsi que des concentrations d'ions, de molécules ou de substances actives.

5. Dispositif selon l'une quelconque des revendications précédentes, **caractérisé en ce que**, pour une augmentation fonctionnelle du nombre et de la précision de séparation des lieux de stimulation pouvant être atteints de manière sélective, les signaux d'impulsions (S₁, S₂, S₃) peuvent, pour un nombre donné de microcontacts (6) implantés de manière stationnaire, être dirigés aussi bien sur des microcontacts individuels (16, 17, 18), que sur plusieurs de tels microcontacts qui sont localement voisins, et **en ce que** les fonctions de temps de stimulation qui, du fait des ordres du codeur, sont définies individuellement avec précision pour chaque microcontact (16, 17, 18) et sont décodées de manière correspondante dans l'interface implantée (12), en particulier en ce qui concerne l'évolution dans le temps, l'intensité du courant, la polarité et la position de phase, ont de leur côté pour effet des évolutions dans le temps caractéristiques et des répartitions locales de lieux du champ électromagnétique dans la zone des neurones à stimuler.

6. Dispositif selon l'une quelconque des revendications précédentes, **caractérisé en ce que** les évolutions dans le temps et les répartitions de lieux, lesquelles sont sélectionnées ou sont déterminées par un procédé d'adaptation et concernent les répartitions de champs qui sont provoqués sur plusieurs microcontacts (16, 17, 18) par une superposition de différents signaux de stimulation ajustés entre eux, déclenchent en tant que foyers de stimulation (F, F'), localement et sélectivement dans le temps, des excitations d'impulsions neuronaux.

7. Dispositif selon l'une quelconque des revendications précédentes, **caractérisé en ce que**, pour effectuer un déplacement local du foyer de stimulation (F, F'), les lieux de stimulation sélectifs (F, F') peuvent être rapidement changés par une variation appropriée des signaux d'impulsions (S₁, S₂, S₃) superposés.

8. Dispositif selon l'une quelconque des revendications précédentes, **caractérisé en ce qu'**il est prévu un système de surveillance pour une structure implantée agissant partiellement de manière sensorimotrice et servant à surveiller et à évaluer divers signaux enregistrés, ainsi qu'à contrôler des effets qui ont lieu à l'endroit de l'implantation et qui sont produits par la structure implantée (1, 6, 7 ; 22, 24, 26) qui communique de manière bidirectionnelle avec le codeur, la structure implantée (1, 6, 7 ; 22, 24, 26) disposant de divers capteurs (9, 22), de processus chimiques d'analyse et de réaction, ainsi que d'actionneurs (26) pour exercer des actions locales mécaniques, électriques, physiques autres, ainsi que chimiques sur le lieu d'implantation et d'une commande sensorimotrice adaptative (1, 24) en communication avec le codeur.

9. Dispositif selon l'une quelconque des revendications précédentes, **caractérisé en ce que** les microcontacts implantés (6, 7, 22) sont utilisables par l'intermédiaire d'une communication bidirectionnelle aussi bien pour la stimulation que pour l'enregistrement d'impulsions neuronales et/ou **en ce qu'**en outre des microcontacts pour l'enregistrement d'une activité neuronale, ainsi que de paramètres physiques, tels que par exemple la pression ou la température, et de concentrations d'ions ou de molécules dans la structure implantée (1, 6, 7 ; 22, 24, 26) sont disponibles.

10. Implant spinal adaptatif pour atténuer des perturbations fonctionnelles neuronales dans la moelle épinière ou dans le système nerveux périphérique, en particulier en ce qui concerne la motricité des parties d'extrémité, le trajet urogénital et les sensations non souhaitables de douleurs ou les impression fantomatiques, avec un codeur adaptatif, une structure implantée (1, 6) qui agit de façon partiellement autonome et de manière sensorimotrice et un système bidirectionnel de transmission de signaux et d'énergie (2, 12, 13), le codeur étant en contact bidirectionnel avec des parties de la moelle épinière, du système nerveux périphérique ou de groupes de muscles.

11. Implant crânien adaptatif pour atténuer des perturbations fonctionnelles neuronales du système nerveux central avec des effets sensoriels, moteurs ou cognitifs non souhaités, y compris des perturbations de la mémoire et des changements de personnalité, avec un codeur adaptatif, avec une unité centrale de contrôle pour des fonctions de traitement de signaux, pour des fonctions de surveillance, pour des fonctions de commande et/ou pour des fonctions d'intervention externe d'une structure implantée partiellement autonome, agissant de manière sensorimotrice (22, 24, 26) et disposant de microcontact (16, 17, 18, 22), de détecteurs, d'un dépôt de substances actives et d'un applicateur de substances actives (26), le codeur étant en contact bidirectionnel avec des régions cérébrales (21) à l'intérieur du crâne par l'intermédiaire d'un système de transmission de signaux et d'énergie (24).

12. Dispositif selon l'une quelconque des revendications précédentes, **caractérisé en ce qu'**un système de surveillance réalise de manière partiellement autonome, par l'intermédiaire d'un système de commande adaptatif existant à cet endroit, une évaluation des signaux enregistrés sur le lieu d'implantation et les transforme en ordres d'intervention locaux physiques ou chimiques.

13. Dispositif selon l'une quelconque des revendications précédentes, **caractérisé en ce que** des fonctions correspondantes du codeur adaptatif (3) sont utilisées pour une adaptation fonctionnelle basée sur la perception, réalisée de manière automatique ou commandée par l'extérieur, aussi bien de filtres sensoriels spatiotemporels pour une optimisation des sensations que de filtres spatiotemporels motorisés ou d'autres modules de commande pour l'optimisation du déroulement de mouvements, pour une application de substances actives commandée par les besoins ou pour des interventions microchirurgicales ou se rapportant à des soins réparateurs.

14. Dispositif selon l'une quelconque des revendications précédentes, **caractérisé en ce que**, dans le cas d'implants spinaux, il existe des systèmes de mesure (5) pour les mouvements des yeux et de la tête ou d'autres articulations pour permettre une commande des fonctions des implants et pour permettre de signaler les intentions ou les perceptions du porteur de l'implant et **en ce qu'**il est possible que des procédés correspondants pour la sélection de différents déroulements de fonctions par une unité d'entrée d'ordres intégrée ainsi que pour une transmission en continu à l'aide d'un transmetteur de signaux approprié intégré de paramètres d'état sélectionnés se rapportant au porteur de l'implant et à l'implant à un organe sensoriel adapté, par exemple le sens du toucher ou un soigneur soient utilisés et soient mis en oeuvre en étant adaptés au patient respectif.

15. Dispositif selon l'une quelconque des revendications précédentes, **caractérisé en ce qu'**il est prévu un système de commande adaptatif pour l'évaluation des signaux enregistrés sur le lieu d'implantation, y compris des analyses chimiques et la production de réactions chimiques à l'intérieur de la structure implantée, et **en ce que** l'évaluation est transformée en ordres d'intervention physiques ou chimiques locaux, comme par exemple une stimulation électrique locale, une application de substances actives ou une intervention thermique pour des objectifs de traitement chirurgical ou se rapportant à des soins réparateurs.

16. Dispositif selon l'une quelconque des revendications précédentes, **caractérisé en ce que** des dépôts locaux de substances actives implantés (26) sont activés en fonction d'une zone d'état patho-physiologique pour délivrer de manière localement limitée et synchronisée dans le temps des quantités de substances actives dosées, laquelle zone d'état est détectée par l'intermédiaire de détecteurs (22) qui sont implantés de manière appropriée et qui servent par exemple pour détecter une activité neuronale ou de cellules gliales, des paramètres physiques, ainsi que des concentrations d'ions, de molécules ou de substances actives, et laquelle zone d'état est évaluée lors d'une phase adaptative se rapportant aux besoins d'action, et **en ce que** des fonctions partielles de ce dégagement local et basé sur l'état de substances actives peuvent être commandées de manière autonome dans la structure implantée ou en communication bidirectionnelle avec le codeur, et ceci à l'extérieur du crâne directement par le porteur de l'implant et/ou par un soigneur.
